(19) Europäisches Patentamt
European Patent Office
Office européen des brevets

(11) Publication number: 0 183 451 B1

(12) # EUROPEAN PATENT SPECIFICATION

(45) Date of publication of the patent specification: **24.08.88**

(51) Int. Cl.⁴: **C 07 C 29/16**, B 01 J 23/46

(21) Application number: **85308340.0**

(22) Date of filing: **15.11.85**

(54) **Catalyst for producing alcohols from olefins and synthesis gas.**

(30) Priority: **26.11.84 FI 844634**

(43) Date of publication of application: **04.06.86 Bulletin 86/23**

(45) Publication of the grant of the patent: **24.08.88 Bulletin 88/34**

(84) Designated Contracting States: **DE FR GB NL**

(56) References cited:
**EP-A-0 014 225**
**US-A-4 144 191**

(73) Proprietor: **NESTE OY, Keilaniemi, SF-02150 Espoo 15 (FI)**

(72) Inventor: **Alvila, Leila, Sepankatu 15 B 26, 80110 Joensuu (FI)**
Inventor: **Pakkanen, Tapani, Koulukatu 34 A 12, 80120 Joensuu (FI)**
Inventor: **Krause, Outi, Kaunispaantie 3 F 59, 00970 Helsinki (FI)**
Inventor: **Joutsimo, Matteus, Riskutie 25 A, 00950 Helsinki (FI)**

(74) Representative: **Lamb, John Baxter et al, MARKS & CLERK 57/60 Lincoln's Inn Fields, London WC2A 3LS (GB)**

EP 0 183 451 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The present invention concerns a catalyst with the aid of which alcohols can be produced using the so-called hydroformylating reaction.

Hydroformylating is understood to be a reaction in which aldehydes are formed of olefins and synthesis gas ($H_2$ + CO). The obtained aldehydes are frequently hydrated to become alcohols:

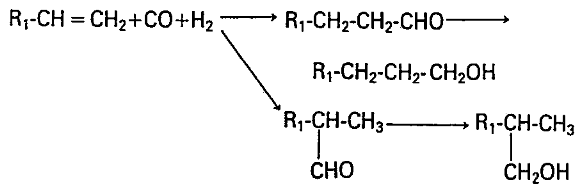

The process was invented by O. Roelen in Germany in the year 1938.

The starting materials may be aither pure olefin or a mixture of olefins. Those olefins react most rapidly which have a double bond at the end of the carbon chain ($\alpha$-olefins), next rapidly, other olefins with straight chain, and slowest, the olefins with branched chain. With the composition of the starting mixture it is possible to exert an effect on the isomer distribution of the product mixture.

In industrial processes, homogeneous catalysts are employed. The process conditions are strongly dependent on the catalyst. Typical catalysts are cobalt hydrocarbonyl ($HCo(CO)_4$, cobalt carbonyl modified with trialkylphosphine, e.g. $(Co(CO)_3P(C_4H_9)_3)_2$, and rhodium carbonyl modified with triphenylphosphine $HRh(CO)(PPh_3)_3$.

In the U.S. Patent No. 4144191, a bimetallic cluster catalyst is disclosed for producing single-step production of alcohols by hydroformylation. The cluster compound was the rhodium-cobalt cluster $Rh_xCo_yCO_{12}$, where x and y are integers between 1 and 3, and $\varepsilon(x+y) = 4$. The compound may be prepared, for instance, according to the following reaction equation:

$$3Co_2(CO)_8 + 2Rh_2(CO)_4Cl_2 \rightarrow 2Co_2Rh_2(CO)_{12} + 2CoCl_2 + 8CO$$

The resulting bimetal cluster is higly air-sensitive and therefore requires very precise handling. As taught by said patent, when the momental clusters $Rh_4(CO)_{12}$ and $Co_4(CO)_{12}$ were separately used, the forming of alcohols decreased substantially. The mixture of monometal clusters $Rh_4(CO)_{14}$ and $Co_2(CO)_8$ produced aldehydes to a large part.

As taught by the present invention, the mixture of monometal cluster compounds $Rh_4(CO)_{12}$ and $Co_4(CO)_{12}$ acted as a highly selective catalyst in producing alcohols. The cluster mixture catalyst behaved in the same way as the bimetal cluster compound, whereas it is rather much simpler to prepare the cluster mixture than the complex bimetal cluster.

Homogeneous hydroformylation nowadays includes six steps:

(1) hydroformylation

(2) removal of the catalyst from the reaction mixture

(3) regeneration of the catalyst

(4) purification of the aldehyde

(5) hydration

(6) distillation of the alcohol

If the process can be accomplished with the aid of a solid catalyst without aldehyde intermediate step, the number of process steps can be reduced to two:

(1) hydroformylation

(2) distillation of the alcohol.

Said procedure considerably simplifies the hydroformylation process.

It is possible to heterogenize the cluster compounds onto the surfaces of various catalyst carriers. Typical carriers are aluminium oxide and silicon dioxide, zeolites, and ion exchange resins. Ion exchange resins containing amines have proved most advantageous.

The cluster compounds can be applied on the surface of the carrier eg. by impregnation. Typically, carrier and a metal cluster compound are mixed in a solvent carefully for at least 16 hrs. Thereafter, the solvent is decanted and the catalyst is dried in vacuum. The catalyst is immediately ready for use.

The activity of the mixture catalyst of monometal clusters was tested in batch reactor tests. The typical reaction conditions were as follows: catalyst quantity 0.01 to 0.2 g, temperature 330 to 490 °K pressure 2 to 5 MPa, and reaction time 1 to 20 hrs.

It was observed in the tests that the metal ratio of the monometal cluster and the amount thereof had an influence on the conversion, selectivity and the product distribution of the alcohols. For example in the variation range Rh:Co = 1.0–2.0 of the molar ratio the conversion of the olefin constituting the starting material was then about 98% and the selectivity of alcohol formation 95%. The aldehyde quantity was typically less than 1%. In the alcohols, the proportion of alcohols with branched chains and those with straight chain ranged from 1.0 to 1.5. When $\alpha$-olefin was used for starting material, three different alcohol isomers were formed thereof:

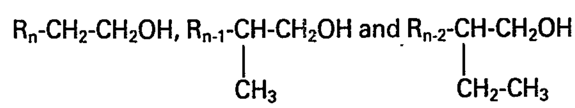

Example

Production of catalyst

30 mg $Co_4(CO)_{12}$ (Strem Chemicals Inc.), 57 mg $Rh_4(CO)_{12}$ [Martinengo, S. et al. Inorg. Synth. 20 (1980) 209], 125 mg Dowex® MWA-1 and 10 ml nitrogenated toluens were mixed in nitrogen atmosphere for 18 hours. The toluence containing non-bound clusters was removed and the catalyst was dried in vacuum.

Hydroformylation

1 ml 1-hexene, 3 ml toluene and 80 mg catalyst were transferred in a nitrogen atmosphere into an

autoclave into which 2.5 MPa $H_2$ and 2.5 MPa CO were added. The autoclave was kept overnight at about 370 °K. After cooling, the product mixture was analyzed by IR spectrometry and capillary gas chromatography. The reaction product contained 90% alcohol. The selectivity of formation of alcohols was 95%.

**Claims**

1. A catalyst for producing alcohols from olefins and synthesis gas, characterized in that it is produced from a mixture of the monometal clusters $M_4(CO)_{12}$ and $M'_4(CO)_{12}$, wherein M and M' belong to the group cobaltmetals, and is bound onto a catalyst carrier.
2. Catalyst according to claim 1, characterized in that M and M' are selected from the group consisting of cobalt, rhodium and iridium.
3. Catalyst according to claim 1 or 2, characterized in that the molar ratio between the monometal clusters is between 1 and 3.
4. Catalyst according to claims 1–3, characterized in that the rhodium and cobald contents are between 0.1 and 15% by weight.
5. Catalyst according to any of claims 1–4, characterized in that the catalyst carrier is a basic ion exchange resin.
6. Process for producing alcohols from olefins and synthesis gas, characterized in that for catalyst is used a mixture of the monometal compounds $(M_4(CO)_{12}$ and $M'(CO)_{12}$ wherein M and M' are selected from the group belonging to the cobalt metals, which mixture is placed on a catalyst carrier.

**Patentansprüche**

1. Katalysator zur Herstellung von Alkoholen aus Olefinen und Synthesegas, dadurch gekennzeichnet, dass er aus einer Mischung der Monometallcluster $(M_4(CO)_{12}$ und $M'_4(CO)_{12}$, worin M und M' zu der Gruppe Cobaltmetalle gehören, hergestellt und auf einen Katalysatorträger gebunden ist.
2. Katalysator nach Anspruch 1, dadurch gekennzeichnet, dass M und M' aus der Gruppe ausgewählt sind, die aus Cobalt, Rhodium und Iridium besteht.
3. Katalysator nach Anspruch 1 oder 2, dadurch gekennzeichnet, dass das Molverhältnis zwischen den Monometallclustern zwischen 1 und 3 liegt.
4. Katalysator nach einem der Ansprüche 1 bis 3, dadurch gekennzeichnet, dass die Rhodium- und Cobaltgehalte zwischen 0,1 und 15 Masse% liegen.
5. Katalysator nach einem der Ansprüche 1 bis 4, dadurch gekennzeichnet, dass der Katalysatorträger ein basisches Ionenaustauscherharz ist.
6. Verfahren zur Herstellung von Alkoholen aus Olefinen und Synthesegas, dadurch gekennzeichnet, dass als Katalysator eine Mischung der Monometallverbindungen $(M_4(CO)_{12}$ und $M'_4(CO)_{12}$, worin M und M' aus der Gruppe ausgewählt sind, die zu den Cobaltmetallen gehört, verwendet wird, wobei diese Mischung auf einen Katalysatorträger aufgebracht ist.

**Revendications**

1. Catalyseur pour préparer des alcools à partir d'oléfines et de gaz de synthèse, caractérisé en ce qu'il est préparé à partir d'un mélange des conglomérats monométalliques $M_4(CO)_{12}$ et $M'_4(CO)_{12}$, dans lesquels M et M' appartiennent aux métaux du groupe du cobalt, et sont liés sur un support de catalyseur.
2. Catalyseur suivant la revendication 1, caractérisé en ce que M et M' sont choisis dans le groupe constitué du cobalt, du rhodium et d'iridium.
3. Catalyseur suivant les revendications 1 ou 2, caractérisé en ce que le rapport molaire entre les conglomérats monométalliques est entre 1 et 3.
4. Catalyseur suivant les revendications 1 à 3, caractérisé en ce que les teneurs en rhodium et cobalt sont comprises entre 0,1 et 15% en poids.
5. Catalyseur suivant l'une quelconque des revendications 1 à 4, caractérisé en ce que le support du catalyseur est une résine échangeuse d'ions basique.
6. Procédé de préparation d'alcools à partir d'oléfines et de gaz de synthèse, caractérisé en ce qu'on utilise comme catalyseur un mélange des composés monométalliques $M_4(CO)_{12}$ et $M'_4(CO)_{12}$, dans lesquels M et M' sont choisis parmi les métaux du groupe du cobalt, lequel mélange est placé sur un support de catalyseur.